# EUROPEAN PATENT APPLICATION

(11) **EP 4 834 391 A1**
(43) Date of publication of application: **04.11.2026**
(21) Application number: 25173735.9
(22) Date of filing: 30.04.2025
(51) Int. Cl.: A61M 5/20, A61M 5/24, A61M 5/32

(54) **LOADING MECHANISM FOR A DRUG DELIVERY DEVICE**

(71) Applicant: Ypsomed AG, 3401 Burgdorf (CH)
(72) Inventor: Wegner, Stephan, 4051 Basel (CH); Bernhard, Mario, 3400 Burgdorf (CH); Loser, Peter, 3037 Herrenschwanden (CH); Kuhlen, Daniel, 3067 Boll (CH); McDougal, Gavin Fraser, Edinburgh, EH11 2AW (GB)

(57) **Abstract**

The present invention relates to a loading mechanism for a drug delivery device for dispensing a liquid drug from a reservoir through a needle, the loading mechanism comprising a drive unit and a reservoir unit adapted to hold the reservoir and releasably attachable to the drive unit. The drive unit comprises a drive unit housing defining a longitudinal axis, a plunger rod movable inside the drive unit housing in a dispensing direction to dispense the liquid drug and a mechanical drive, preferably a compression spring, arranged inside the drive unit housing for moving, when loaded, the plunger rod in the dispensing direction. The drive unit further comprises a puller that is movable in the proximal direction to pull the plunger rod in the proximal direction to load the mechanical drive. According to the invention the drive unit comprises a lock element that is movable between a first position in which the lock element blocks a distal opening of the drive unit housing and a second position in which the distal opening of the drive unit housing is open such that the reservoir unit is insertable into the distal opening of the drive unit housing, wherein the lock element is adapted to be moved from the first position to the second position when the puller moves in the proximal direction.

## Description

### FIELD OF THE INVENTION

The present invention relates to drug delivery devices or medicament delivery devices for injecting, delivering, administering, infusing or dispensing substances and/or liquids such as insulin, hormone preparations or vaccines. It departs from a drug delivery device comprising a preferably reusable drive unit and a preferably disposable reservoir unit configured for releasable attachment to the drive unit.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a stopper in a container barrel, wherein the drive spring may have to be charged or strained manually prior to injection of a dose. A manually powered delivery drive requires a user to manually provide the energy to move the stopper, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the stopper manually or by pressing a release button of an automatic injection device. Automatic injection devices may comprise an electronic dose dial mechanism to automatically set a dose.

Also known is the use of autoinjectors with syringes. Autoinjectors usually comprise a body for housing a syringe as well as a drive mechanism to move the stopper of the syringe upon actuation of the autoinjector. The drive mechanism typically comprises a source of drive, such as an electric motor or a mechanical spring for moving a transfer element, for example a plunger rod, which acts on the stopper of the syringe. The prefilled syringe has a needle or cannula that is permanently attached to a first end of a syringe barrel or reservoir that is sealed at the opposing end by the stopper.

For safety and hygiene reasons it is desirable that the needle does not protrude from the housing of the autoinjector except for the time when the needle is used for injection of a medicament. Thus, either the autoinjector moves the needle out of the housing for the injection and back into the housing after injection or the autoinjector includes a needle cover which may be moved to unsheathe the needle for injection, and which may be moved back to a needle covering position after the injection.

The majority of autoinjectors are configured as single use devices which incorporate both the syringe and the drive mechanism in the same housing. Such devices are usually disposable for hygiene reasons. Disposable autoinjectors comprising an electric actuator or an electronic control require a source of energy which is usually in the form of a battery. However, in this case, the autoinjectors should not be disposed of in the regular waste but must be subjected to special disposal or to recycling, which is an additional burden to the patient. Further, disposing of a battery, motor and/or electronics after a single use is a waste of resources and increases the costs of the therapy. Disposable autoinjectors comprising a mechanical drive, like a compression spring, are easier to dispose of, however it is also a waste of resources to dispose of the compression spring after a single use, which also increases the costs of the therapy.

To account for the need to handle and dispose of the autoinjector parts differently, semi-reusable autoinjectors have been developed. Such autoinjectors typically comprise a reusable drive unit and a disposable syringe unit which may be releasably coupled or attached to the drive unit. The drive unit usually includes the drive mechanism and electronics whereas the syringe unit includes the syringe with the needle and a needle cover sleeve. The user can thus discard the syringe unit when it is empty or after use and can load the drive unit with a new syringe unit for a new upcoming injection. A big problem for semi-reusable mechanical autoinjectors is that the compression spring must be recompressed or re-loaded after each injection, which can require a high force and accuracy, which can be challenging for the user, especially for older patients. A related problem is that users might not remember if they have already re-loaded the injection spring. However, it can be dangerous if the user tries to insert the syringe unit when the spring is not loaded, due to the spring repulsing the syringe unit when the spring is only partly re-loaded.

US 2006/069354 A1 discloses a semi-reusable autoinjector with a reusable drive unit comprising a drive spring and a syringe that is insertable into the drive unit. To re-load the drive spring, the drive unit comprises a piston retraction yoke that a user can pull in a proximal direction away from the injection side of the drive unit. After the drive spring is loaded, the syringe is laterally insertable into the drive unit through a hinged clamshell door. For the injection, the piston rod then pushes the syringe towards the desired injection site so that the needle is inserted into the skin of the user and consequently the medicament is dispensed by further movement of the plunger. US 2011/0172602 A1 discloses a similar injection device.

These known semi-reusable autoinjectors are sometimes difficult to assemble and there is a risk that the syringe is inserted in a twisted or turned orientation so that the plunger rod exerts the drive force on the syringe piston in a non-perpendicular direction, which can destroy the syringe or lead to an amount of injected medicament that deviates from the desired amount. Another general problem is that it is dangerous for the user and can lead to injuries of the user if the user tries to insert the syringe before the retraction yoke is pulled back.

### DESCRIPTION OF THE INVENTION

It is an objective of the present invention to provide a drug delivery device with a mechanical drive that allows a user to load the drug delivery device by tensioning the mechanical drive and to insert the disposable reservoir in an easy and reliable way before each injection.

This objective is achieved by the features of the independent claims. Preferred embodiments are evident from the dependent claims.

The invention relates to a loading mechanism for a drug delivery device for dispensing a liquid drug from a reservoir through a needle.

The loading mechanism comprises a drive unit and a reservoir unit adapted to hold the reservoir and releasably attachable or connectable to the drive unit. With the loading mechanism, a mechanical drive of the drive unit can be loaded, and the reservoir unit can be inserted into the drive unit before each injection. The drug delivery device is preferably an injection device and more preferably an autoinjector, that is adapted to dispense the whole content of the reservoir in a single injection stroke. Most preferably, the drug delivery device is a semi-disposable autoinjector with a reusable drive unit and a disposable reservoir unit. After each injection, the empty reservoir unit is disposed and replaced by a new disposable reservoir unit. The reservoir may be a prefilled syringe having a needle or cannula that is permanently attached to a first end of a syringe barrel that is sealed at the opposing end by a stopper. Alternatively, the reservoir may be a cartridge with a connecting portion at the distal end adapted to be connected to an injection needle before the injection.

The drive unit of the loading mechanism comprises a drive unit housing defining a longitudinal axis and a plunger rod movable inside the drive unit housing in a dispensing direction to dispense the liquid drug. The plunger rod can also be referred to as piston rod or drive element. The longitudinal axis defines a longitudinal direction of the drive unit. The longitudinal axis of the drive unit is also considered as the longitudinal axis of the drug delivery device. The dispensing direction can preferably be the longitudinal direction and more preferably a distal direction. The drive unit housing can comprise only one part with a sleeve structure. The drive unit housing can also comprise two or more parts that together form a sleeve structure. A separation between the parts can run in the circumferential direction of the sleeve, in other words the parts can be sleeves with a shorter length, or transversely thereto, in other words the parts can be half shells or partial shells which together form a sleeve structure. In this alternative, the two or more drive unit housing parts can be connected mechanically, for example with screws or latches, or they can be bonded to each other, for example by gluing or welding. The drive unit further comprises a mechanical drive arranged inside the drive unit housing for moving, when loaded with the loading mechanism, the plunger rod in the dispensing direction. Preferably, the mechanical drive is a compression spring. For loading, the mechanical drive is preferably compressed in a proximal direction, hence a distal end of the mechanical drive is moved in the proximal direction while the proximal end of the mechanical drive is fixed.

The reservoir unit can comprise a reservoir unit housing, the reservoir, a needle cover, and a protective cap to remove a needle shield of the reservoir. Preferably, the reservoir is fixed or non-movably arranged inside the reservoir unit housing or a reservoir holder arranged inside the reservoir unit housing. That means that during an injection the reservoir and hence the needle does not move relative to the reservoir unit housing or the reservoir holder and hence also does not move relative to the drive unit housing. This means that the user must insert or penetrate the injection needle into the injection site by a manual insertion force.

The drive unit of the loading mechanism further comprises a puller that is movable in the proximal direction to pull or move the plunger rod in the proximal direction to load the mechanical drive. A distal portion of the puller can be telescopically received in the drive unit housing or may telescopically surround the drive unit housing, so that when the puller is moved in the proximal direction, the puller protrudes further from the proximal end of the drive unit housing. Preferably the puller can move back into its initial position when the user releases the puller. This can for example be achieved by a puller spring. Preferably the plunger rod is latched in its proximal position, in which the mechanical drive or drive spring is compressed, so that the mechanical drive stays in the compressed position when the user releases the puller, making the medicament delivery device ready to perform an injection. The puller can for example comprise a ring feature and/or ergonomically formed recesses and/or protrusions to make it easier for the user to grab and move the puller and to exert the force needed to load the mechanical drive.

According to the invention, the drive unit of the loading mechanism further comprises a lock element that is movable between a first position in which the lock element blocks a distal opening of the drive unit housing and a second position in which the distal opening of the drive unit housing is open such that the reservoir unit is insertable into the distal opening of the drive unit housing. The lock element is adapted to be moved from the first position to the second position when the puller moves in the proximal direction. Preferably the reservoir unit is inserted into the drive unit housing by a user prior to an injection to assemble the drug delivery device and to prepare the drug delivery device for the injection. Hence, the inserting of the reservoir unit into the drive unit housing does not take place during assembly of the drug delivery device or any other manufacturing step by the manufacturer, before the reservoir unit and the drive unit are delivered to the user. With the loading mechanism according to the invention, the reservoir unit can only be inserted after the puller is pulled in the proximal direction which means that the mechanical drive is loaded. Before the puller is moved in the proximal direction, the lock element blocks the opening of the drive unit housing so that the reservoir unit cannot be inserted. This means that with the inventive loading mechanism, in a first step the mechanical drive is loaded and in a second step the reservoir unit is inserted into the drive unit housing. Preferably, the lock element can additionally be used to latch the plunger rod. Alternatively, the plunger rod could be latched by flexible plunger rod clips that latch to the drive unit housing.

An advantage of the loading mechanism according to the invention is that the force needed to insert the reservoir unit is small, because the mechanical drive is already loaded by pulling on the plunger rod so that no additional force is needed to load the mechanical drive during the insertion of the reservoir unit. This two-step loading mechanism in which loading of the mechanical drive and inserting of the reservoir unit is separated, makes it particularly easy and comfortable for the user to insert the reservoir unit. Another advantage is that the lock element prevents the user from inserting the reservoir unit before the mechanical drive is loaded in a way that is easy to understand and easy to perform. Inserting the reservoir unit without previously loading the mechanical drive could lead to the plunger rod pushing the stopper of the reservoir further inside the reservoir and thereby expelling medicament from the reservoir before the actual injection. Therefore, the loading mechanism provides a safe and uncomplicated way to prepare the semi-reusable medicament delivery device for an injection.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The term "distal" is meant to refer to the direction or the end of the drug delivery device carrying an injection needle or an injection cannula, whereas the term "proximal" is meant to refer to the opposite direction or end pointing away from the needle or cannula.

The term "injection device" or "injector" refers to a device that is removed from the injection site after each medication event or drug delivery process, whereas the term "infusion device" refers to a device with a cannula or needle that remains in the skin of the patient for a prolonged period of time, for example, several hours.

In the claims, the word "comprising" or "comprises" does not exclude other elements or steps, and the indefinite article "a" or "an" does not exclude a plurality. For example, "an arm" does not exclude the fact that there may be two arms that functionally or structurally fulfill the purpose of "an arm". The mere fact that certain elements or steps are recited in distinct claims shall not preclude the existence of further meaningful combinations of these elements or steps.

In a preferred embodiment, the lock element is rotatable between the first position and the second position. In this preferred embodiment, the lock element can be adapted to be rotatable but fixed axially within the drive unit housing.

In a preferred embodiment, the reservoir unit is insertable into the drive unit housing from the distal end of the drive unit housing by a linear movement along the longitudinal axis, hence in a longitudinal direction. Preferably, the reservoir unit can also be separated from the drive unit housing by a linear movement along the longitudinal axis. This makes it particularly easy for the user to understand how to insert or remove the reservoir unit and to actually insert or remove the reservoir unit.

In a preferred embodiment, the reservoir unit comprises a reservoir unit housing comprising a viewing window and the lock element is adapted to cover the viewing window in the first position and to reveal the viewing window in the second position. This allows the user to easily see whether the mechanical drive is loaded and hence whether the reservoir unit can be inserted.

In a preferred embodiment, the lock element is biased towards the first position by a lock spring, which preferably is a torsion spring. With the lock spring the lock element returns to the first position when the plunger rod has passed the lock element. This can latch the plunger rod and thereby keep the mechanical drive in the compressed state even if the user releases the puller.

In a preferred embodiment, the puller is biased in the distal direction by a puller spring, which preferably is an extension spring. The puller spring is connected with one end to the puller and with the other end to the drive unit housing. This reduces the user steps that the user needs to do as the puller returns automatically to its initial position and is ready to be used again after the injection is completed. The puller spring preferably biases the puller back into the housing when the user releases the puller.

In a preferred embodiment, the lock element is movable, preferably rotatable, to a third position when the puller has reached a proximal end position. The third position is located between the first position and the second position. In the third position preferably, the plunger rod is latched by the lock element so that it cannot move in the distal direction releasing the loaded mechanical drive and the distal opening of the drive unit is open to allow insertion of the reservoir unit.

In a preferred embodiment, the plunger rod comprises a plunger rod projection with an angled proximal surface. In this preferred embodiment the lock element slides along the proximal angled surface when the plunger rod moves in the proximal direction, so that the lock element rotates from the first position to the second position. When the lock element rotates, the plunger rod projection can pass the lock element so that the plunger rod can move in the proximal direction to load the mechanical drive. Preferably, the lock element can comprise a rib element on the inside of the lock element that slides over the angled proximal surface of the plunger rod projection. This provides a compact and easy design in which the lock element at least partially surrounds the plunger rod.

Preferably, the plunger rod projection comprises a lateral recess in a distal end surface of the plunger rod projection and the lock element comprises a rib. The rib of the lock element is received in the lateral recess of the plunger rod projection when the plunger rod projection has passed the rib. When the rib is received in the lateral recess, the lock element is in the third position. Preferably, the lock element is moved or rotated into the third position under the bias of the lock spring. The rib is then received in the lateral recess which prevents the plunger rod from moving axially in case the lock element is axially fixed within the drive unit housing.

Preferably, the puller comprises an end block abutting a distal end of the plunger rod projection and a puller arm connected to the end block and extending parallel to the plunger rod. Preferably, the puller can comprise two or more puller arms, preferably arranged in equal distances to each other in a circumferential direction, wherein the plunger rod is arranged between the two or more puller arms. This provides an easy design for the puller and for the plunger rod that also allows to reduce the radial dimension of the drug delivery device.

In a preferred embodiment, the reservoir unit comprises a needle cover or cover member, wherein the needle cover is movable between an extended position in which it covers the needle and a retracted position in which it reveals the needle. Preferably, the needle cover is movable along the longitudinal axis and/or guided by the reservoir unit housing. Preferably, the needle cover can move from the extended position to the retracted position by pressing the medicament delivery device and hence the needle cover against the skin at a desired injection site which pushes the needle cover into the reservoir unit and optionally into the drive unit. Preferably, the drive unit can comprise a needle cover pusher or pusher sleeve that abuts the needle cover and moves together with the needle cover. This allows the use of a shorter needle cover and makes it easier to use the movement of the needle cover to release the plunger rod. The needle cover is adapted to move the lock ring from the third position to the second position to release the plunger rod, when the needle cover reaches the retracted position. Preferably, the needle cover is a needle cover sleeve. The movement of the needle cover allows switching between a safety state in which the needle is covered and does not protrude from the reservoir unit and an injection state in which the needle is uncovered and protrudes from the reservoir unit. Additionally, in this preferred embodiment, the needle cover is used as release element to release the plunger rod, so that no extra elements, such as a release button or injection button, and no extra user steps, such as pressing an additional release button or injection button, are necessary. Preferably, the needle cover can be biased towards the extended position, for example with a needle cover spring. Preferably, after the injection the needle cover can be biased into a final extended position in which the needle cover is locked and can hence not move back into the reservoir unit or further out of the reservoir unit to provide needle protection. The final extended position can preferably be more distal than the initial extended position.

In a preferred embodiment, in a proximal end surface of the reservoir unit housing a recess is formed, that comprises a proximal angled portion with a decreasing width from a proximal end to a distal end and a distal lock portion wider than the distal end of the angled portion. In this preferred embodiment, the drive unit comprises a spring biased button biased in a radial direction. The button comprises a latching element that is adapted to slide over the proximal angled portion while the reservoir unit is inserted into the drive unit housing. This moves the button against the bias of the button spring to be received in the lock portion when the reservoir unit reaches a mounting position within the drive unit housing to establish a connection between the reservoir unit and the drive unit. In the mounting position, the reservoir unit is fixedly attached or connected to the drive unit. The mounting position can be understood as a predefined end position of the reservoir unit within the drive unit housing. The recess and the spring biased button must be adapted to be able to provide the connection between the reservoir unit and the drive unit against the force of the loaded mechanical drive and eventually against the force of removing a protective cap from the reservoir unit after attaching the reservoir unit to the drive unit and before the injection. The latching element can preferably be a pin, which can preferably have an angled distal surface to make the insertion of the reservoir unit easier. This preferred embodiment can also be used for other drug delivery devices not according to this invention; hence this preferred embodiment is not necessarily dependent on the aspects and preferred embodiments previously described.

Preferably, the connection between the drive unit and the reservoir unit is separable by pushing the spring biased button against the bias of the spring. This allows the user to remove the reservoir unit, as the connection between the reservoir unit and the drive unit is separated or released. The needle cover preferably comprises a protrusion that is adapted to prevent movement of the spring biased button in the retracted position of the needle cover. With other words, the protrusion on the needle cover blocks the movement of the latching element of the spring biased button and hence the spring biased button. Hence the reservoir unit cannot be removed during the injection when the needle cover is in the retracted position. This prevents accidental release of the reservoir unit during the injection. Preferably, the projection on the needle cover has an angled surface to reduce friction, which reduces the risk of the needle cover not returning to its extended position when the user keeps pressing the button.

In a preferred embodiment, the reservoir unit comprises a cap attachable to the reservoir unit housing. The cap comprises a locking clip with a locking protrusion received in a recess in the reservoir unit housing in an initial position, so that the cap is locked to the reservoir unit housing. In this preferred embodiment, the drive unit housing comprises an unlocking rib that deflects the locking clip in the radial direction moving the locking protrusion out of the recess, when the reservoir unit reaches the mounting position, so that the cap can be removed from the reservoir unit. With other words, the cap is locked to the reservoir unit housing until the reservoir unit is fully inserted. This has the advantage that the user cannot accidentally touch the needle while inserting the reservoir unit into the drive unit. The unlocking rib can be located on an inner surface of the drive unit housing so that it deflects the locking clips radially inwards when the reservoir unit is fully inserted. To allow the deflection of the locking clips, the needle cover can comprise a clearance cut in an outer surface of the needle cover into which the locking clips can deflect. Alternatively, the unlocking rib can be located on an outer surface of the drive unit housing so that it deflects the locking clip radially outwards when the reservoir unit is fully inserted. In both alternatives, when unlocked, the cap can preferably be pulled off the reservoir unit housing in a straight axial direction. This preferred embodiment makes it easy to deflect the locking clips to be able to pull off the cap, as the user does not have to deflect the locking clips directly, for example by pressing on the locking clips with a finger. This preferred embodiment can also be used for other drug delivery devices not according to this invention; hence this preferred embodiment is not necessarily dependent on the aspects and preferred embodiments previously described.

The invention also relates to a drug delivery device comprising an inventive loading mechanism. Preferably, the reservoir unit housing, the drive unit housing, the plunger rod, the puller, the lock element, the needle cover, the spring biased button and/or the cap are injection-molded thermoplastic plastic parts. The injection-molded parts allow for a cost-effective production of the drug delivery device parts in particular in case of a high-volume production.

### BRIEF DESCRIPTION OF THE DRAWINGS

The subject matter of the invention will be explained in more detail in the following text with reference to preferred example embodiments which are illustrated in the attached drawings, in which depict:
- Fig. 1: an example embodiment of an inventive drug delivery device;
- Fig. 2: the drug delivery device of fig. 1 in an exploded view;
- Fig. 3: the drive mechanism subassembly of the drug delivery device of fig. 1 and 2 in an exploded view;
- Fig. 4: the housing subassembly of the drug delivery device of fig. 1 and 2 in an exploded view;
- Fig. 5: an example embodiment of an inventive loading mechanism for the drug delivery device of fig. 1 to 4;
- Fig. 6: a detailed view of the distal end of the drug delivery device of fig. 1 to 4 with the lock element in the first position;
- Fig. 7: a detailed view of the distal end of the drug delivery device of fig. 1 to 4 with the lock element in the second position;
- Fig. 8: a sectional view of the drive unit of the drug delivery device of fig. 1 to 7 with the lock element in the first position;
- Fig. 9: a sectional view of the drive unit of the drug delivery device of fig. 1 to 7 with the lock element in the second position;
- Fig. 10: a sectional view of the drive unit of the drug delivery device of fig. 1 to 7 with the lock element in the third position;
- Fig. 11: a sectional view of the drug delivery device of fig. 1 to 10 while the reservoir unit is inserted into the drive unit;
- Fig. 12: a sectional view of the drug delivery device of fig. 1 to 10 after the reservoir unit is fully inserted into the drive unit;
- Fig. 13: a sectional view of a distal end of the drive unit and the reservoir unit of an embodiment of a drug delivery device according to the invention before the reservoir unit is fully inserted into the drive unit;
- Fig. 14: the distal end of the drive unit and the reservoir unit of fig. 13 after the reservoir unit is fully inserted into the drive unit;
- Fig. 15: a sectional view of a distal end of the drive unit and the reservoir unit of another embodiment of a drug delivery device according to the invention before the reservoir unit is fully inserted into the drive unit;
- Fig. 16: the distal end of the drive unit and the reservoir unit of fig. 15 after the reservoir unit is fully inserted into the drive unit;
- Fig. 17: a schematic view of the drug delivery device of fig. 1 to 12 with the lock element in the third position;
- Fig. 18: a schematic view of the drug delivery device of fig. 1 to 12 with the lock element in the second position;
- Fig. 19: a sectional view of a distal part of the drug delivery device of fig. 1 to 12; and
- Fig. 20: a second example embodiment of a drug delivery device according to the invention.

The reference symbols used in the drawings, and their primary meanings, are listed in summary form in the list of designations. In principle, identical parts are provided with the same reference symbols in the figures.

### DETAILED DESCRIPTION OF THE PREFFERRED EMBODIMENTS

In the present description the term "distal" refers to the side where the injection needle is located and the term "proximal" refers to the opposite side or rear end of the device.

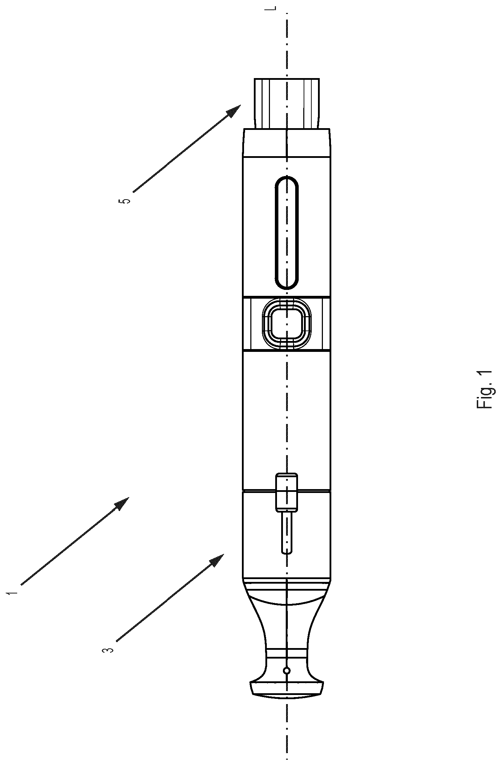

Fig. 1 shows an example embodiment of a drug delivery device 1 of the present disclosure in form of a semi-reusable autoinjector. Autoinjector 1 comprises a reusable drive unit 3 and a disposable reservoir unit 5 insertable in the drive unit 3. The reservoir unit 5 is insertable into drive unit 3 from a distal end of the drive unit 3 by a linear movement in a longitudinal direction along a longitudinal axis L defined by the drive unit 3.

The drive unit 3 of this example embodiment has a length of 195.5 mm in the longitudinal direction and a slightly elliptical cross section with outer dimensions of 30 mm and 28 mm. The reservoir unit 5 of the example embodiment has a length of 90.9 mm and a rectangular cross section with rounded edges and outer dimensions of 22 mm and 16.5 mm. The assembled autoinjector 1 as shown in fig. 1 has a total length of 213 mm, hence a major part of the reservoir unit 5 is inserted into the drive unit 3. This allows for a compact design of autoinjector 1.

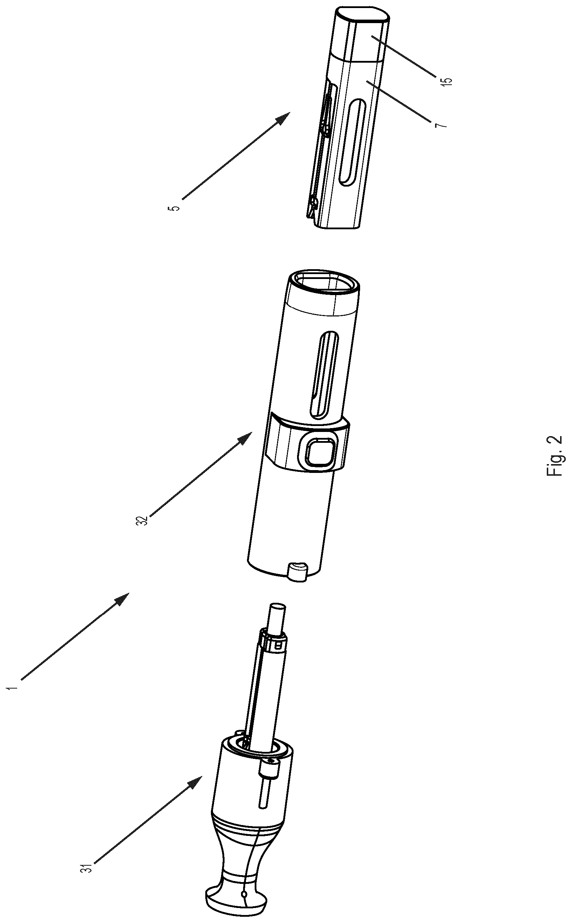

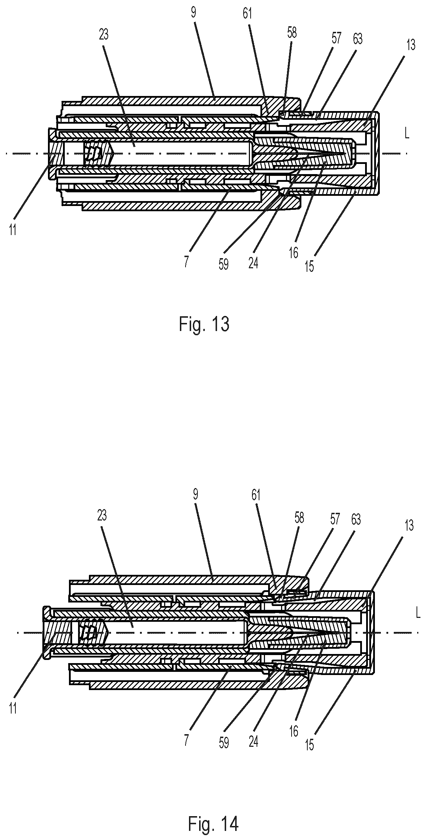

Fig. 2 shows the autoinjector 1 of fig. 1 in exploded view. In this embodiment, the drive unit 3 comprises a drive mechanism subassembly 31 and a housing subassembly 32. In other embodiments, the drive mechanism subassembly and the housing assembly can also be integrated in only one housing part. The reservoir unit 5 of the autoinjector 1 comprises a reservoir unit housing 7, a needle cover 13 (not visible in fig. 1, see for example fig. 13 to 18), a cap 15 and a reservoir 23 with a needle 24 (not visible in fig. 1, see for example fig. 13 to 18). The reservoir is a prefilled syringe or cartridge with a stopper at a proximal end and a needle or cannula at a distal end. The reservoir may be adapted to be connected to the needle or cannula before an injection or the needle or cannula may be permanently attached to the reservoir. The reservoir further comprises a needle shield 16 (not visible in fig. 1, see for example fig. 13 to 18) that covers and protects the needle before the injection.

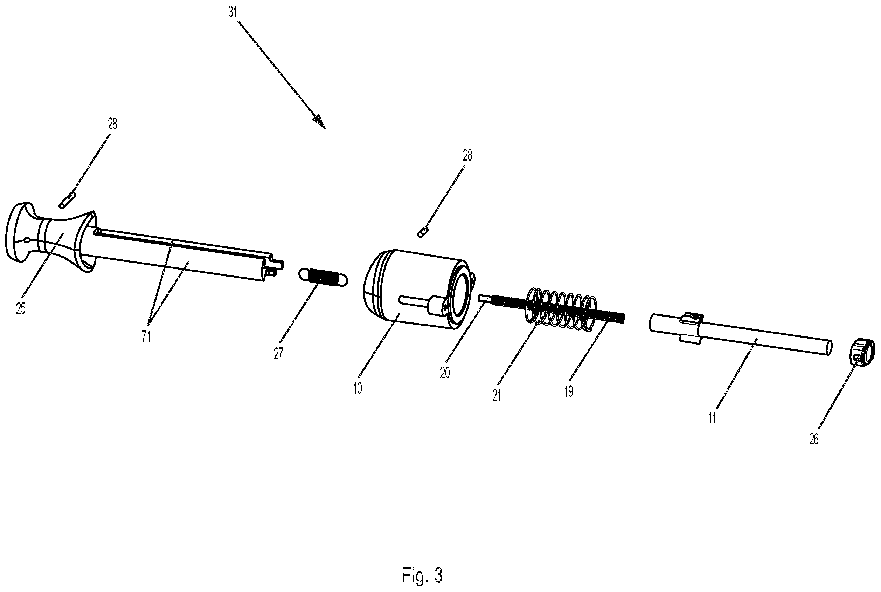

Fig. 3 shows an exploded view of the drive mechanism subassembly 31. The main components of the drive mechanism subassembly 31 and hence of the drive unit 3 are a drive unit housing cap 10 that is part of a drive unit housing 9, a plunger rod 11, a mechanical drive 19 in form of a compression spring, a pusher sleeve spring 21 also in form of a compression spring, a puller 25 and a puller spring 27 in the form of an extension spring. The puller 25 comprises an end block 26 and two puller arms 71 that will be explained in detail below. The puller spring 27 is connected with one end to the puller 25 and with the other end to the housing cap 10 and biases the puller 25 in the distal direction towards its distal position. In this embodiment, the connection is provided by pins 28. The drive mechanism subassembly 31 of the drive unit 3 also comprises a mechanical drive rod 20 that is arranged inside the mechanical drive 19 and prevents the mechanical drive 19 from bending during compression and decompression of the mechanical drive 19.

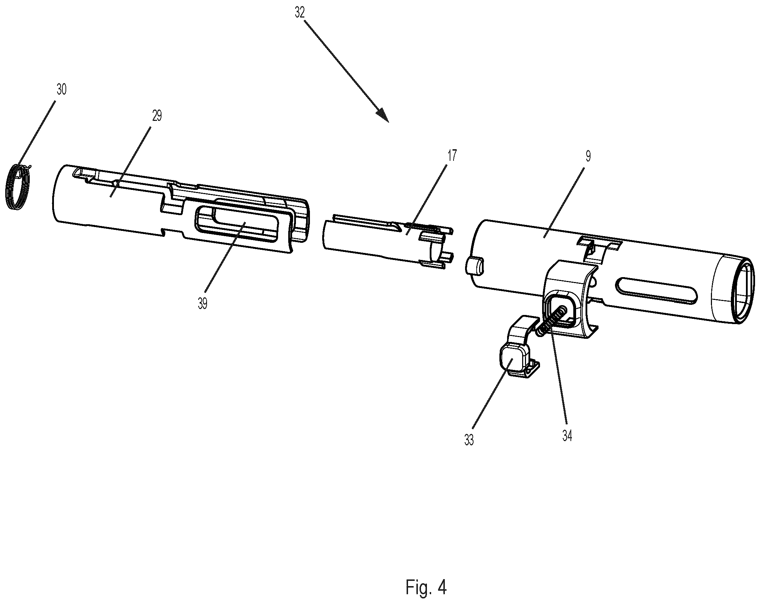

Fig. 4 shows an exploded view of the housing subassembly 32. The main components of the housing subassembly 32 and hence of the drive unit 3 are a drive unit housing 9, a pusher sleeve 17 and a lock element 29. In this embodiment, lock element 29 is a lock sleeve. Further, the housing subassembly 32 of the drive unit 3 comprises a spring biased button 33 biased by a button spring 34, that allows the user to remove the reservoir unit 5 from the drive unit 3, and a lock spring 30 in form of a torsion spring. In the embodiment of fig. 1 to 4, the drive unit housing cap 10 is attachable to the drive unit housing 9 by a screw and nut connection and corresponding screw holes in the drive unit housing 9 and the drive unit housing cap 10. In embodiments in which the drive unit 3 does not comprise subassemblies, the drive unit housing and the drive unit housing cap can be formed as one integral part respectively the drive unit housing cap 10 can be integrated into the drive unit housing 9.

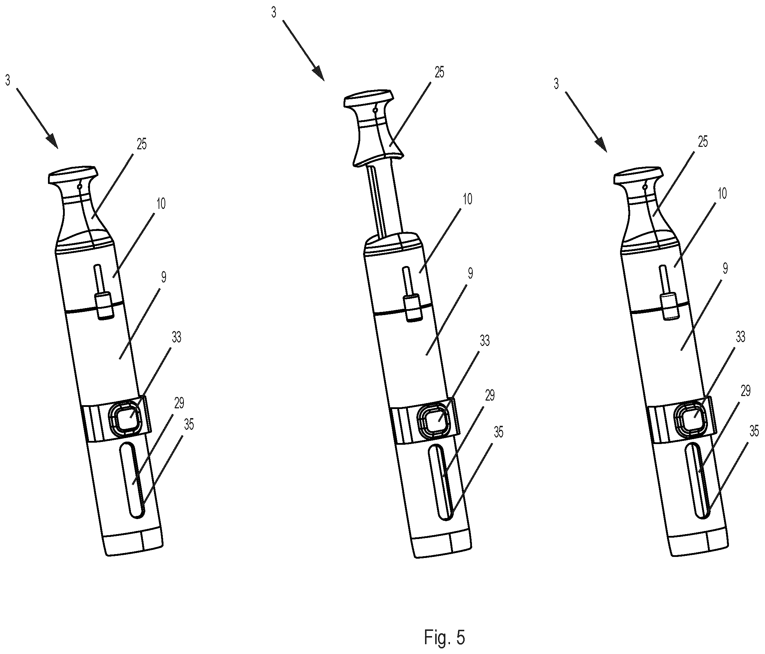

Fig. 5 shows an example embodiment of an inventive loading mechanism for the drug delivery device of fig. 1 to 4 that allows to load the mechanical drive. In an initial state shown on the left of fig.5, the puller 25 is in its distal position and the mechanical drive is relaxed. The lock element 29 is in a first position in which it covers a viewing window 35 in the drive unit housing 9, so that the interior of the drive unit 3 is not visible. In the first position, lock element 29 also blocks a distal opening of the drive unit housing 9 for the insertion of the reservoir unit into the drive unit 3. When the user moves the puller 25 in the proximal direction, as shown in the middle in fig. 5, puller 25 pulls the plunger rod 11 in the proximal direction to load the mechanical drive. The proximal movement of the puller 25 also rotates lock element 29 to a second position in which lock element 29 no longer covers the viewing window 35 and no longer blocks the distal opening of the drive unit housing 9. The plunger rod 11 is latched in its proximal position, so that the user can release the puller 25 to return to its distal position as shown on the right in fig. 5. To latch the plunger rod 11 in its distal position, the lock element 29 moves into a third position. In the third position, lock element 29 does not rotate all the way back to the first position so that the viewing window 35 and the distal opening are still open. Hence, the third position of the lock element 29 is between the first position and the second position of the lock element 29. The user can then insert the reservoir unit into the drive unit 3.

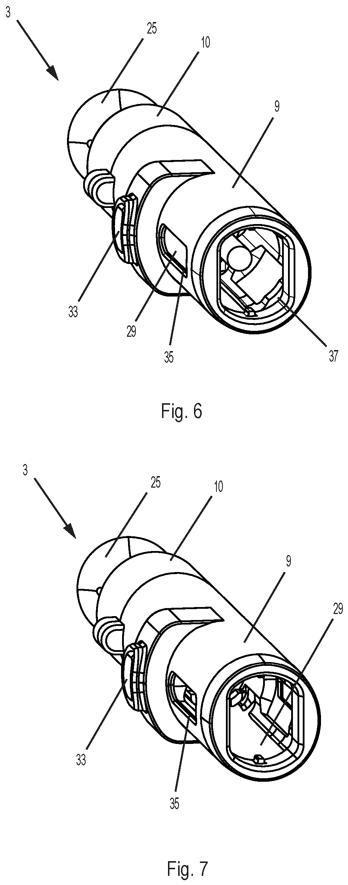

The movement and the function of the locking element 29 to cover and open the distal opening is now explained in detail with reference to fig. 6 and 7, wherein fig. 6 shows the distal end of the drug delivery device 1 with the lock element 29 in the first position and fig. 7 shows the distal end of the drug delivery device 1 with the lock element 29 in the second position. The lock element 29 comprises a flange 37 that blocks the opening in the first position and is rotated out of the opening in the second position, so that the opening is no longer blocked. To achieve this, the distal opening of the drive unit housing 9 has a rectangular cross-section. To block and open the viewing window 35, lock element 29 comprises a recess 39 (see fig. 4) that is located next to the viewing window 35 in the first position and beneath the viewing window 35 in the second position, so that viewing window 35 is no longer covered by the sidewall of the lock element 29.

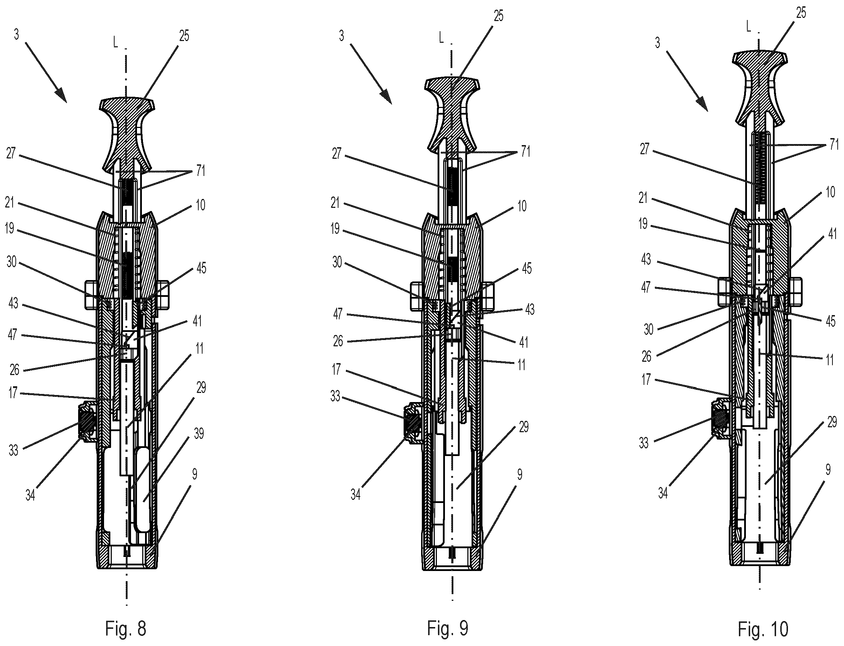

Fig. 8 to 10 show the drive unit 3 in a sectional view, to illustrate the cooperation between the puller 25, the lock element 29 and the plunger rod 11, wherein in fig. 8 the lock element 29 is in the first position, in fig. 9 the lock element 29 is in the second position and in fig, 10 the lock element 29 is in the third position between the first position and the second position.

In fig. 8, the puller 25 is in its distal position so that the puller spring 27 is relaxed. The plunger rod 11 is also in its distal position so that the mechanical drive 19 is also relaxed. The lock element 29 is in the first position in which it blocks the distal opening of the drive unit housing 9 and covers the viewing window 35 as explained before. The end block 26 of the puller 25 is arranged distal from a circumferential plunger rod projection 41 of the plunger rod 11.

When the puller 25 is moved in the proximal direction, the plunger rod 11 is also pulled in the proximal direction by the end block 26 abutting the plunger rod projection 41. The puller arms 71 are connected to the end block 26 and therefore move the end block 26 together with the proximal part of the puller 25 that is pulled by the user. As shown in fig. 9, by the proximal movement of the puller 25, the puller spring 27 and the mechanical drive 19 are both partly loaded in that the puller spring 27 is extended and the mechanical drive 19 is compressed. The plunger rod projection 41 comprises an angled proximal surface 43 that leads to a rotation of the lock element 29 from the first position to the second position, when the puller 25 is moved in the proximal direction. The angled proximal surface 43 contacts a rib 45 on an inner surface of the lock element 29 so that the rib 45 slides over the angled proximal surface 43 when the puller 25 is moved further in the proximal direction.

Due to the rotation of lock element 29 into the second position, the plunger rod projection 41 can pass the rib 45 so that the puller 25 can be moved to its proximal end position as shown in fig. 10. When the puller 25 reaches the proximal end position, the puller spring 27 and the mechanical drive 19 are fully loaded. The rotation of the lock element 29 from the first position to the second position additionally tensions the lock spring 30 that biases the lock element 29 back towards the first position. The plunger rod projection 41 further comprises a lateral recess 47 on a distal end of the plunger rod projection 41. When the plunger rod projection 41 has passed the rib 45, the lock spring 27 pushes the rib 45 into the recess 47, so that the lock element 29 does not move all the way back to the first position but stays in the third position with the rib 45 in the recess 47 as shown in fig. 10. The recess 47 is closed towards the proximal end, so that the rib 45 now prevents the plunger rod 11 from moving back in the distal direction and thereby latches the plunger rod 11 in its proximal position in which the mechanical drive 19 is fully loaded. Due to the loaded puller spring 27, the puller 25 returns to its distal position, when the user lets go off the puller 25.

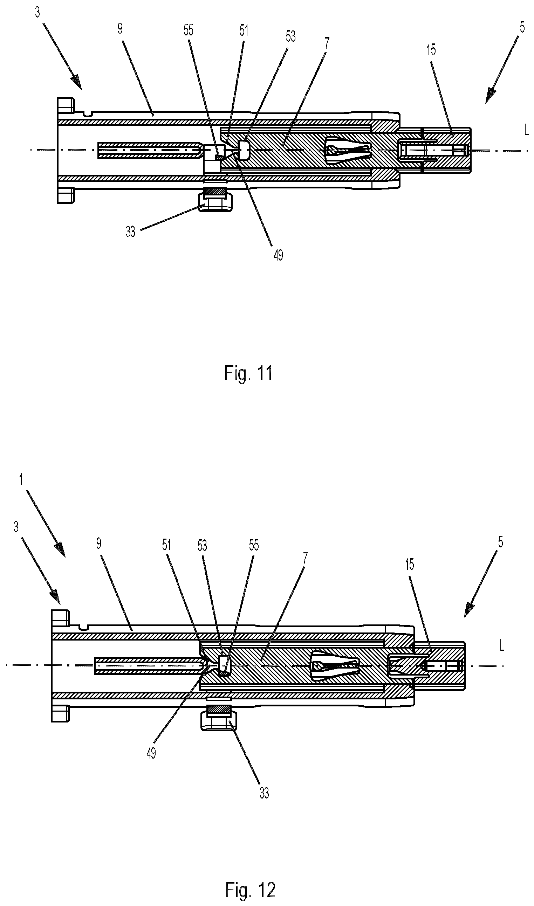

Fig. 11 and 12 illustrate how the reservoir unit 5 is inserted into and fixed or latched inside the drive unit 3, wherein fig. 11 shows a cross-sectional view of the autoinjector 1 while the reservoir unit 5 is inserted into the drive unit 3 and fig. 12 shows a cross-sectional view of the autoinjector 1 after the reservoir unit 5 is fully inserted into the drive unit 3. The reservoir unit 5 is inserted into the drive unit 3 from a distal end of the drive unit 3 by pushing the reservoir unit 5 into the drive unit housing (not shown in fig. 11 and 12). To latch the reservoir unit 5 inside the drive unit 3, the reservoir unit housing 7 comprises a recess 49 in a proximal end surface of the reservoir unit housing 7 that cooperates with the spring biased button 33 of the drive unit 3. The recess 49 comprises a proximal angled portion 51 with a decreasing width from a proximal end to a distal end and a distal lock portion 53 wider than the distal end of the angled portion 51. The spring biased button 33 comprises a latching element 55 in the form of a pin arranged on a surface of the button 33 that faces the reservoir unit 5. The latching element 55 slides over the proximal angled portion 51 while the reservoir unit 5 is inserted into the drive unit housing 9 as shown in fig. 11 to move the button 33 against the bias of the button spring 34 (not shown in fig. 11) and thereby loading the button spring 34. When the reservoir unit 5 is fully inserted and reaches a mounting position as shown in fig. 12, the latching element 55 is pushed into lock portion 53 by the button spring so that the latching element 55 is received in lock portion 53. Thereby, the button 33 and the recess 49 in the reservoir unit housing 7 establish a connection between the reservoir unit 5 and the drive unit 3. To release this connection after the injection is complete, the user can press the button 33 to move the latching element 55 out of the lock portion 53.

Fig. 13 and 14 illustrate the removal of cap 15 of reservoir unit 5 before the injection can be started. The cap 15 is engaged with the needle shield 16 of the reservoir 23, so that when the cap15 is removed, the needle shield 16 is removed together with the cap 15. Fig. 13 shows a distal end of the drive unit 3 and the reservoir unit 5 of an inventive drug delivery device 1 before the reservoir unit 5 is fully inserted into the drive unit 3 and fig. 14 after the reservoir unit 5 is fully inserted into the drive unit 5.

In Fig. 13, the cap 15 is locked to the reservoir unit housing 7. In the embodiment of fig. 13 and 14, the locking is achieved by locking clips 57 with locking protrusions 58 on a proximal end of the cap 15 and recesses 59 at a distal end of the reservoir unit housing 7 that receive the locking protrusions 58, so that cap 15 cannot move in the distal direction. Cap 15 is unlocked, when the reservoir unit 5 is fully inserted into the drive unit 3. In the embodiment of fig. 13 and 14, the unlocking is achieved by unlocking ribs 61 on an inner surface of the drive unit housing 9. When the locking clips 57 reach the unlocking ribs 61, the unlocking ribs 61 push the locking clips 57 radially inward moving the locking protrusions 58 out of the recesses 59, so that the cap 15 can then be removed from the reservoir unit 5 in the distal direction. Hence, the cap 15 is locked to the reservoir unit 5 until the reservoir unit 5 is fully inserted into the drive unit 3. To enable the radial inwards movement of the locking clips 57, the needle cover 13 comprises clearance cuts 63 in an outer surface of the needle cover 13 into which the locking clips 57 can move.

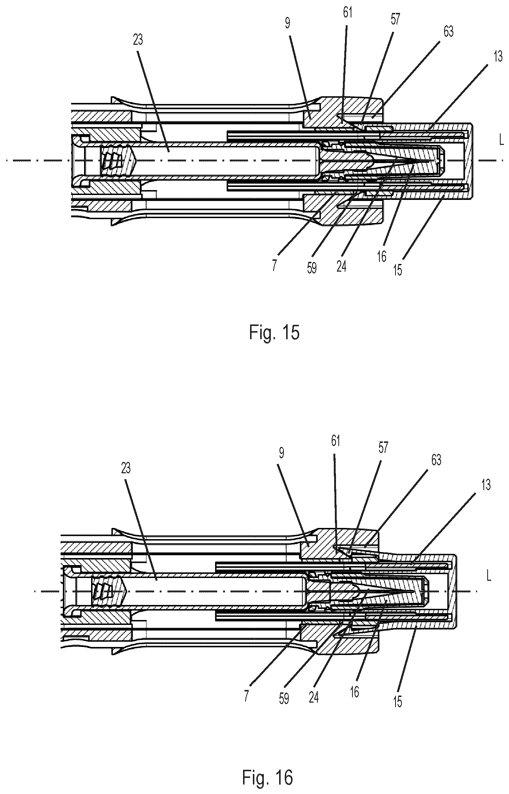

Fig. 15 and 16 illustrate another possibility for the locking and removal of cap 15. In this embodiment, the unlocking rib 61 pushes the locking clips 57 radially outwards to move the locking protrusions 58 out of the recesses 59. Therefore, the clearance cut 63 is formed in the drive unit housing 9.

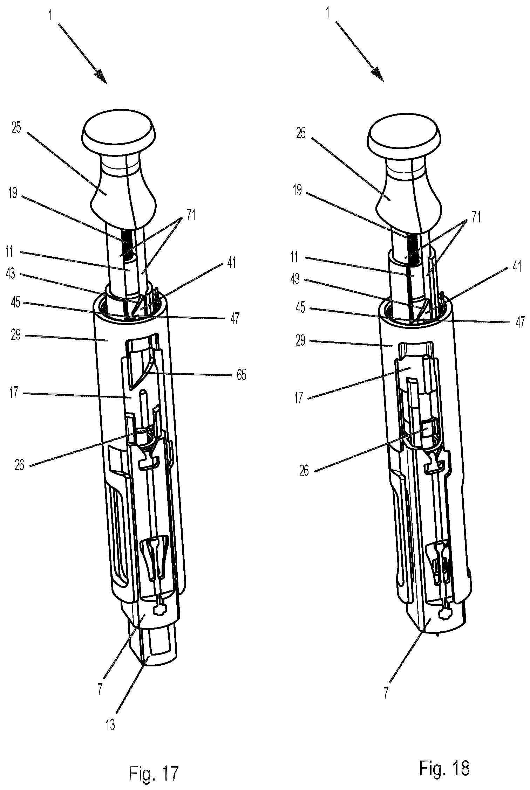

Fig. 17 and 18 illustrate how the injection process can be started, wherein fig. 17 shows autoinjector 1 before the injection is triggered with lock element 29 in the third position and fig. 18 shows the autoinjector 1 after the injection is triggered with lock element 29 in the second position. The drive unit housing is omitted in fig. 17 and 18 to better show the interior of the autoinjector 1.

The injection is triggered by pressing the autoinjector 1 against the skin at a desired injection site. This moves the needle cover 13 from the extended position in which it covers the needle 24 to a retracted position in which the needle cover 13 is retracted into the reservoir unit housing 7 and the needle 24 is revealed. With other words the needle cover 13 is pushed into the reservoir unit housing 7. This proximal movement of the needle cover 13 also pushes the pusher sleeve 17 in the proximal direction. The pusher sleeve 17 comprises a ramp feature 65 that faces the rib 45 of the lock element 29. When the needle cover 13 and therefore also the pusher sleeve 17 reaches the retracted position, the ramp feature 65 contacts the rib 45 and rotates the lock element 29 from the second position shown in fig. 17 to the third position in fig. 18. This moves the rib 45 out of the lateral recess 47 of the plunger rod projection 41, which unlatches or releases the plunger rod 11 so that the plunger rod 11 is free to move in the distal direction under the bias of the loaded mechanical drive 19. The plunger rod 11 then pushes the stopper of reservoir 23 in the distal direction to expel the medicament from the reservoir 23. After the injection, the autoinjector can be removed from the skin which releases the needle cover 13 to move back to its distal position under the bias of the pusher sleeve spring 21 that biases the pusher sleeve 17 and the needle cover 13 in the distal direction. In the distal position, the needle cover 13 fully covers the needle 24. Preferably, the needle cover 13 is locked in the distal position after the injection, so that it is not possible to push it back into the reservoir unit housing 7. This prevents the user from accidentally touching the needle after the injection is complete.

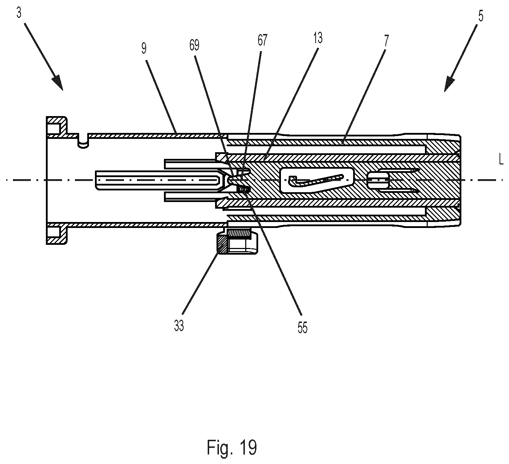

After the injection, the user can eject the reservoir unit 5 out of the drive unit 3 by pressing the spring biased button 33. When the spring biased button 33 is pressed, the latching element 55 moves out of the distal lock portion 53 of the reservoir unit housing 7 so that the reservoir unit 5 is free to move in the distal direction out of the drive unit 3. The ejection of the reservoir unit 5 can be aided by a residual force in the pusher sleeve spring 21. Fig. 19 illustrates a feature that allows to prevent accidental release of the reservoir unit 5, for example during the injection, by accidentally pressing or touching the spring biased button 33. For this purpose, the needle cover 13 comprises a projection 67 on its proximal end. During the injection, when the needle cover 13 is in the retracted position, the projection 67 blocks the latching feature 55 from moving out of the distal lock portion 53 and prevents the spring biased button 33 from being pushed in. Projection 67 has ramped proximal surfaces 69 to reduce friction between the projection and the latching element 55, which reduces the risk of the needle cover 13 not moving in the distal direction to its extended position, when the spring biased button 33 is held.

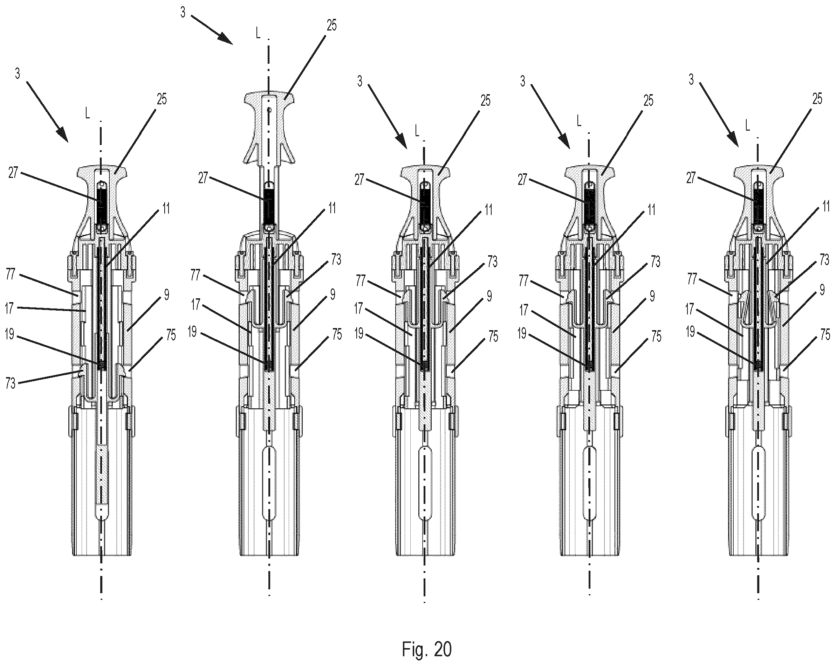

Fig. 20 shows a second example embodiment of a drug delivery device 1 according to the invention, in the form of a semi-reusable autoinjector. The second example embodiment differs from the first example embodiment in fig. 1 to 12 in the way the plunger rod 11 is latched and moved by the puller 25. Apart from these features, the second example embodiment essentially corresponds to the first example embodiment, so that the same reference numbers are used for common features.

In the embodiment of fig. 20, the plunger rod 11 comprises two plunger rod clips 73 that can be received in first recesses 75 and in second recesses 77 of the drive unit housing 9 to latch the plunger rod 11 in two distinct positions. When the plunger rod clips 73 are received in the first recesses 75, as shown on the left in fig. 20, the plunger rod 11 is in its most distal position and the mechanical drive 19 of the autoinjector 1 is unloaded respectively relaxed. When the user then pulls the puller 25 in the proximal direction, the puller 11 moves together with the puller 25 so that the plunger rod clips 73 are pushed out of the first recesses 75 due to ramped proximal surfaces of the plunger rod clips 73. The plunger rod clips 73 are then received in the second recesses 77 when the plunger rod 11 and the puller 25 reach their most proximal position, so that the plunger rod 11 is latched in its most distal position in which the mechanical drive 19 is loaded. This position is shown in the second image from the left of fig. 20. To translate the movement of the puller 25 into a movement of the plunger rod 11, the puller 25 contacts a distal end surface of the plunger rod clips 73.

When the user releases the puller 25, the puller returns to its distal position under the bias of the puller spring 27, while the plunger rod 11 stays in the proximal position due to the plunger rod clips 73. This position is shown in the middle of fig. 20. The user then presses autoinjector 1 against the skin as shown in the second image from the right in fig. 20. This retracts the needle cover 13 (not shown in fig. 20). The needle cover pushes thereby pushes a pusher sleeve 17 in the proximal direction, which deflects the plunger rod clips 73 inwards and out of the second recesses 77, when the needle cover reaches its most proximal position. This is shown on the right of fig. 20. When the plunger rod clips 73 are deflected, the plunger rod 11 is no longer latched and is therefore moved in the distal direction by the force of the loaded mechanical drive 19 to dispense the medicament.

The previous description of the disclosed embodiments is provided to enable a person skilled in the art to make or use the disclosed embodiments. Various modifications to these embodiments will be readily apparent to those skilled in the art, and the principles defined herein may be applied to other embodiments without departing from the scope of the disclosure. Thus, the present disclosure is not intended to be limited to the embodiments shown herein but is to be accorded the widest scope possible consistent with the principles and novel features as previously shown and described.

### LIST OF DESIGNATIONS

- 1: drug delivery device
- 3: drive unit
- 5: reservoir unit
- 7: reservoir unit housing
- 9: drive unit housing
- 10: drive unit housing cap
- 11: plunger rod
- 13: needle cover
- 15: cap
- 16: needle shield
- 17: pusher sleeve
- 19: mechanical drive
- 20: mechanical drive rod
- 21: pusher sleeve spring
- 23: reservoir
- 24: needle
- 25: puller
- 26: end block
- 27: puller spring
- 28: pin
- 29: lock element
- 30: lock spring
- 31: drive mechanism subassembly
- 32: housing subassembly
- 33: spring biased button
- 34: button spring
- 35: viewing window
- 37: lock element flange
- 39: recess
- 41: plunger rod projection
- 43: angled proximal surface
- 45: rib
- 47: lateral recess
- 49: recess
- 51: proximal angled portion
- 53: distal lock portion
- 55: latching element
- 57: locking clip
- 58: locking protrusion
- 59: recess
- 61: unlocking rib
- 63: clearance cut
- 65: ramp feature
- 67: projection
- 69: ramped proximal surface
- 71: puller arm
- 73: plunger rod clip
- 75: first recess
- 77: second recess

- L: longitudinal axis

## Claims

1. A loading mechanism for a drug delivery device (1) for dispensing a liquid drug from a reservoir (23) through a needle (24), the loading mechanism comprising a drive unit (3) and a reservoir unit (5) adapted to hold the reservoir (23) and releasably attachable to the drive unit (3),
the drive unit (3) comprising a drive unit housing (9) defining a longitudinal axis (L), a plunger rod (11) movable inside the drive unit housing (9) in a dispensing direction to dispense the liquid drug and a mechanical drive (19), preferably a compression spring, arranged inside the drive unit housing (9) for moving, when loaded, the plunger rod (11) in the dispensing direction,
wherein the drive unit (3) further comprises a puller (25) that is movable in the proximal direction to pull the plunger rod (11) in the proximal direction to load the mechanical drive (19),
**characterized in that** the drive unit (3) comprises a lock element (29) that is movable between a first position in which the lock element (29) blocks a distal opening of the drive unit housing (9) and a second position in which the distal opening of the drive unit housing (9) is open such that the reservoir unit (5) is insertable into the distal opening of the drive unit housing (9), wherein the lock element (29) is adapted to be moved from the first position to the second position when the puller (25) moves in the proximal direction.

2. Loading mechanism according to claim 1, wherein the lock element (29) is rotatable between the first position and the second position.

3. Loading mechanism according to claim 1 or 2, wherein the reservoir unit (5) is insertable into the drive unit housing (9) by a linear movement along the longitudinal axis (L).

4. Loading mechanism according to one of the preceding claims, wherein the reservoir unit (5) comprises a reservoir unit housing (7) comprising a viewing window (35) and the lock element (29) is adapted to cover the viewing window (35) in the first position and to reveal the viewing window (35) in the second position.

5. Loading mechanism according to one of the preceding claims, wherein the lock element (29) is biased towards the first position by a lock spring (30), which preferably is a torsion spring.

6. Loading mechanism according to one of the preceding claims, wherein the puller (25) is biased in the distal direction by a puller spring (27), which preferably is an extension spring, connected with one end to the puller (25) and with the other end to the drive unit housing (9).

7. Loading mechanism according to one of the preceding claims, wherein the lock element (29) is movable, preferably rotatable, to a third position when the puller (25) has reached a proximal end position, wherein the third position is between the first position and the second position.

8. Loading mechanism according to one of the preceding claims, wherein the plunger rod (11) comprises a plunger rod projection (41) with an angled proximal surface (43), wherein the lock element (29) slides along the angled proximal surface (43) when the plunger rod (11) moves in the proximal direction, so that the lock element (29) is rotated from the first position to the second position.

9. Loading mechanism according to claims 7 and 8, wherein the plunger rod projection (41) comprises a lateral recess (47) in a distal end surface of the plunger rod projection (41) and the lock element (29) comprises a rib (45), wherein the rib (45) is received in the lateral recess (47), when the plunger rod projection (41) has passed the rib (45).

10. Loading mechanism according to one of claims 8 or 9, wherein the puller (25) comprises an end block (26) abutting a distal end of the plunger rod projection (41) and a puller arm (71) connected to the end block (26) and extending parallel to the plunger rod (11).

11. Loading mechanism according to one of the preceding claims, wherein the reservoir unit (5) comprises a needle cover (13), wherein the needle cover (13) is movable between an extended position in which it covers the needle (24) and a retracted position in which it reveals the needle (24), wherein the needle cover (13) is adapted to move the lock element (29) from the third position to the second position to release the plunger rod (11), when the needle cover (13) reaches the retracted position.

12. Loading mechanism according to one of the preceding claims, wherein in a proximal end surface of the reservoir unit housing (7) a recess (49) is formed, the recess (49) comprising a proximal angled portion (51) with a decreasing width from a proximal end to a distal end and a distal lock portion (53) wider than the distal end of the angled portion (51), wherein the drive unit (3) comprises a spring biased button (33) biased in a radial direction by a button spring (34), the button (33) comprising a latching element (55) that is adapted to slide over the proximal angled portion (51) while the reservoir unit (5) is inserted into the drive unit housing (9) to move the button (33) against the bias of the button spring (34) and to be received in the lock portion (53) when the reservoir unit (5) reaches a mounting position to establish a connection between the reservoir unit (5) and the drive unit (3).

13. Loading mechanism according to claim 12, wherein the connection between the drive unit (3) and the reservoir unit (5) is separable by pushing the spring biased button (33) against the bias of the button spring (34), wherein the needle cover (13) comprises a protrusion (67) that is adapted to prevent movement of the spring biased button (33) in the retracted position of the needle cover (13).

14. Loading mechanism according to one of the preceding claims, wherein the reservoir unit (5) comprises a cap (15) attachable to the reservoir unit housing (7), the cap (15) comprising a locking clip (57) with a locking protrusion (58) received in a recess (59) in the reservoir unit housing (7) in an initial position, so that the cap (15) is locked to the reservoir unit housing (7), wherein the drive unit housing (9) comprises an unlocking rib (61) that deflects the locking clip (57) in the radial direction moving the locking protrusion (58) out of the recess (59), when the reservoir unit (5) reaches the mounting position, so that the cap (15) can be removed from the reservoir unit (5).

15. A drug delivery device (1) comprising a loading mechanism according to one of claims 1 to 14, wherein in particular the reservoir unit housing (7), the drive unit housing (9), the plunger rod (11), the puller (25), the lock element (29), the needle cover (13), the spring biases button (33) and/or the cap (15) are injection-molded thermoplastic plastic parts.
